# EUROPEAN PATENT APPLICATION

(11) **EP 4 424 258 A1**
(43) Date of publication of application: **04.09.2024**
(21) Application number: 22894791.7
(22) Date of filing: 15.11.2022
(51) Int. Cl.: A61B 18/04

(54) **AUTOMATIC SURGICAL SMOKE EXHAUST SYSTEM**

(30) Priority: 16.11.2021 CN 202111356884
(71) Applicant: CTC Medical Technology (Shandhai) Co., Ltd, Shanghai 201600 (CN)
(72) Inventor: ZHAO, Lei, Beijing 100176 (CN); WEI, Wei, Beijing 100176 (CN); SHAO, Meng, Beijing 100176 (CN); WANG, Xiaohui, Beijing 100716 (CN); ZHANG, Junji, Beijing 100716 (CN); SUN, Dawei, Beijing 100176 (CN)
(74) Representative: Derry, Paul Stefan
(86) International application number: PCT/CN2022/132044
(87) International publication number: WO 2023/088258

(57) **Abstract**

An automatic surgical smoke exhaust system, comprising a smoke exhaust channel (11) and a surgical smoke exhaust device (2). The smoke exhaust channel (11) can guide smoke generated while an energy instrument (12) is working at a surgical site to the surgical smoke exhaust device (2). The surgical smoke exhaust device (2) comprises a smoke exhaust host (21) and an automatic start/stop control unit (22) electrically connected to the smoke exhaust host (21). The smoke exhaust channel (11) is connected to the smoke exhaust host (21) by means of a pipeline (3). The automatic start/stop control unit (22) can be configured to detect a working status of the energy instrument (12), and control start/stop of the smoke exhaust host (21) according to working status information. The automatic surgical smoke exhaust system can achieve automatic control over smoke exhaust, which not only ensures the safety and continuity of a surgery, but also guarantees the health of patients and surgical medical staff.

## Description

### Cross-reference of related applications

The present application claims the benefit of the Chinese invention patent application No. CN202111356884.0, filed on November 16, 2021, the content of which is incorporated herein by reference.

### Field of Technology

The present disclosure relates to the field of medical instruments, and in particular, to an automatic surgical smoke exhaust system.

### Background

With the rapid development of medical technologies, minimally invasive technology has been widely promoted and applied because of its characteristics such as small wounds and easy recovery.

To achieve minimally invasive and non-invasive surgeries, such as a laparoscopic surgery, it is necessary to establish a surgical channel through a puncture outfit in the abdominal wall or even the back of a patient to reduce the damage to the patient. The laparoscopic surgery is a surgical method that is highly dependent on instruments, in which the application of energy instruments such as monopolar and bipolar energy instruments is indispensable in operations such as surgical cutting and hemostasis.

At present, most of the energy instruments use thermal energy or mechanical energy with high-speed and low-frequency oscillation to cause protein denaturation, so that a large amount of smoke will be generated in this process. The problems caused by generation of the smoke are as follows. 1. It affects surgical safety: the large amount of smoke obstructs a surgical field of view, making an operation area blurred, and affecting the safety of surgical operation. 2. It affects surgical continuity and increases the time cost of the surgery. Sometimes, the surgery has to be suspended so that the surgical operation can be continued only after the smoke is dissipated and the surgical field of view is clear. 3. It is potentially harmful to the health of the patient undergoing the surgery as well as doctors and nurses involved in the surgery: the surgical smoke contains a variety of harmful chemical components (organic or inorganic compounds) and even cancerogenic substances. Scholars have definitely found at least four cancerogenic substances in the surgical smoke, i.e., 1,3-butadiene, hydrogen fluoride, acetylene and organic benzene. The smoke may be absorbed by the inner wall of the abdominopelvic cavity of the patient and by the serosa and peritoneum of organ surfaces. Studies have found that significant increase of benzene and methylbenzene are detected in the urine of the patient undergoing the laparoscopic surgery, which is not conducive to post-surgical recovery and even potentially harm to the health of the patient. 4. It is harmful to the health of a surgeon: in addition to the above substances harmful to human bodies or the carcinogenic substances, the surgical smoke may generate a strong odor and infectious biological particles. Studies reported that 1 g of electrosurgically treated human tissue generates smoke that is as harmful as a human body inhaling 6 unfiltered cigarettes. An amount of harmful substances in the smoke from one surgery is equivalent to harmful smoke from 27 to 30 cigarettes. 77% of surgical smoke particles are less than 1.1 um, with an average diameter of only 0.07 um, and also contain some ultrafine particles with an average diameter of 10 nm to 1 um. Despite the tight protection of a surgical mask, particles less than 5 um may be directly inhaled into the lung through the mask. Additionally, harmful substances (i.e., styrene, a teratogen, etc.) may directly enter the body through parts such as eyes to cause damage to the human body.

Therefore, how to effectively exhaust or eliminate the smoke generated by the use of the energy instruments during the surgery and ensure the safety and continuity of the surgery has been an urgent technical problem in the fields of surgery-related researches.

### Summary

The technical problem to be solved by the present disclosure is to provide an automatic surgical smoke exhaust system. The automatic surgical smoke exhaust system can achieve automatic control over smoke exhaust, which can facilitate operation by surgical staff, ensure the safety and continuity of a surgery, and guarantee the health of a patient and the surgical medical staff.

In order to solve the above technical problem, the present disclosure provides an automatic surgical smoke exhaust system, including a smoke exhaust channel and a surgical smoke exhaust device. The smoke exhaust channel can guide smoke generated while an energy instrument is working at a surgical site to the surgical smoke exhaust device. The surgical smoke exhaust device includes a smoke exhaust host, and an automatic start/stop control unit electrically connected to the smoke exhaust host. The smoke exhaust channel is connected to the smoke exhaust host by means of a pipeline. The automatic start/stop control unit can detect a working status of the energy instrument, and control start/stop of the smoke exhaust host according to working status information.

Specifically, the automatic start/stop control unit includes a working status collecting unit and a control unit, wherein the control unit is configured to analyze the working status information fed back by the working status collecting unit and controls the start/stop of the smoke exhaust host.

Specifically, the energy instrument is inserted into the smoke exhaust channel, and a working end of the energy instrument extends out of the smoke exhaust channel.

Optionally, the working status collecting unit is electrically connected between an energy generator and a power supply, and the control unit can obtain a working status of the energy instrument according to transmission power between the power supply and the energy generator.

Preferably, the working status collecting unit is a Hall sensor.

Optionally, the working status collecting unit is an acoustic sensor, so that it is able to detect sound waves intermittently emitted by the energy generator according to a magnitude of output energy power thereof.

Optionally, the working status collecting unit is a light color recognition module, so that it is able to detect indicator light of different colors emitted by a light-emitting unit on the energy generator according to magnitudes of output energy power of the energy generator; alternatively, the working status collecting unit is a light intensity recognition module, so that it is able to detect indicator light of different brightnesses emitted by the light-emitting unit on the energy generator according to magnitudes of output energy power of the energy generator.

Optionally, the working status collecting unit is a temperature sensor, so that it is able to recognize a temperature at an acting area where the energy instrument works at a surgical site.

Optionally, the working status collecting unit is a smoke sensor, so that it is able to recognize a concentration of the smoke generated while the energy instrument is working on the surgical site.

Optionally, the working status collecting unit includes an image acquisition device which can capture the surgical site, and the control unit is embedded with an image processing module so that it is able to recognize smoke in a picture captured by the image acquisition device.

Through the above technical solutions, the present disclosure has the following beneficial effects.

In the automatic surgical smoke exhaust system according to the present disclosure, the energy instrument acting at a surgical site and generating the smoke is inserted into the smoke exhaust channel, and the smoke exhaust channel is connected to the smoke exhaust host by means of the pipeline, so that the smoke generated during the surgery can be sucked, nearby and in time, out from a space of surgery through the smoke exhaust host. The automatic start/stop control unit configured to detect a working status of the energy instrument is provided in the surgical smoke exhaust device, so that an amount of the smoke in the space of surgery can be directly or indirectly obtained according to the working status of the energy instrument, and accordingly start/stop of the smoke exhaust host can be controlled according to the magnitude of the obtained amount of the smoke. In this way, automatic control over smoke exhaust can be achieved, which can facilitate the operation by the surgical staff and make better operation continuity for the surgical staff; and meanwhile, the smoke can be exhausted nearby and in time, the safety and continuity of the surgery are ensured, and the health of the patient and the surgical medical staff is guaranteed.

Other features and advantages of the present disclosure will be described in detail in the subsequent section of Detailed Description of the Embodiments.

### Brief Description of the Drawings

The drawings are used to provide a further understanding of the present disclosure, and constitute a part of the description to illustrate the present disclosure together with the specific embodiments below, but do not constitute a limitation on the present disclosure. In the drawings,
Fig. 1 is a schematic diagram of a structure of Embodiment 1 in the specific embodiments of the present disclosure;
Fig. 2 is a schematic diagram of a structure of Embodiment 2 and Embodiment 3 in the specific embodiments of the present disclosure;
Fig. 3 is a schematic diagram of a structure of Embodiment 4 and Embodiment 5 in the specific embodiments of the present disclosure; and
Fig. 4 is a schematic diagram of a structure of Embodiment 6 in the specific embodiments of the present disclosure.

### Illustrations of reference signs

| | |
|---|---|
| 11 - smoke exhaust channel | 12 - energy instrument |
| 13 - energy generator | 2 - surgical smoke exhaust device |
| 21 - smoke exhaust host | 22 - automatic start/stop control unit |
| 221 - working status collecting unit | 222 - control unit |
| 3 - pipeline | |

### Detailed Description of the Embodiments

The specific embodiments of the present disclosure are described in detail below in conjunction with the drawings. It should be understood that the specific embodiments described herein are intended only to describe and explain the present disclosure rather than to limit the present disclosure.

In the description of the present disclosure, it is to be noted that wordings "mount", "provide", "arrange" and "connect" should be understood in a broad sense, unless otherwise expressly specified and limited. For example, the wording "connect" may refer to a fixed connection, or a detachable connection, or a one-piece connection; it may refer to a direct connection, or an indirect connection via an intermediate medium, or it may be an interior communication between two elements or an interactive relationship between the two elements. For those of ordinary skill in the art, the specific meanings of the above wordings in the present disclosure may be understood according to specific circumstances.

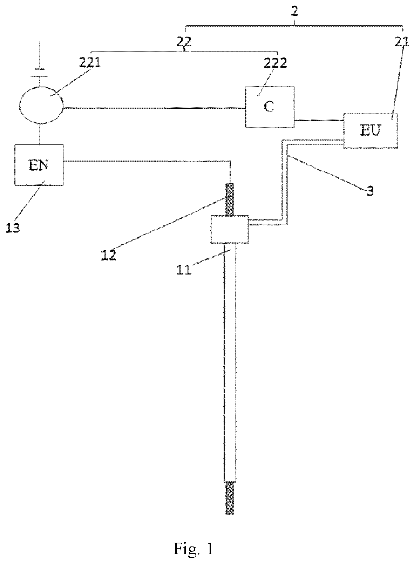

As shown in Fig. 1, an automatic surgical smoke exhaust system according to the present disclosure includes a smoke exhaust channel 11 and a surgical smoke exhaust device 2. The smoke exhaust channel 11 can guide smoke, generated by an energy instrument 12 at a surgical site, to the surgical smoke exhaust device 2. The surgical smoke exhaust device 2 includes a smoke exhaust host 21 and an automatic start/stop control unit 22 electrically connected to the smoke exhaust host 21. The smoke exhaust channel 11 is connected to the smoke exhaust host 21 by means of a pipeline 3. The automatic start/stop control unit 22 can detect a working status of the energy instrument 12, and control start/stop of the smoke exhaust host 21 according to working status information, so that automatic control over smoke exhaust can be achieved.

Specifically, an energy generator 13 is connected to a power supply and can regulate the magnitude of power of the energy instrument 12. The energy instrument 12 may be arranged to be inserted into smoke exhaust channel 11, and a working end of the energy instrument 12 extents out of the smoke exhaust channel 11 so as to perform operations such as cutting on the surgical site. The smoke exhaust channel 11 is connected to the smoke exhaust host 21 by means of the pipeline 3, so that the smoke generated while the energy instrument 12 is working on the surgical site during a surgery can be exhausted out of a space of surgery along the smoke exhaust channel 11, the pipeline 3 and the smoke exhaust host 21 under a suction effect of the smoke exhaust host 21. The pipeline 3 may be provided as a flexible pipeline, so that an entirety formed by the surgical instrument 12 and the smoke exhaust channel 11 can be arranged to be separated from the surgical smoke exhaust device 2, thereby facilitating operation of the surgical instrument 12 within a narrow space of surgery. Preferably, the exhausted smoke is filtered and purified through a smoke storage apparatus or a purification apparatus. Additionally, the automatic start/stop control unit 22 configured to detect the working status of the energy instrument 12 is provided in the surgical smoke exhaust device 2 to directly or indirectly obtained an amount of the smoke in the space of surgery or infer whether the smoke is presented according to the working status of the energy generator 12, so that the start/stop of the smoke exhaust host 21 can be controlled according to the magnitude of the amount of the smoke obtained or the presence of the smoke. In this way, automatic control over smoke exhaust can be achieved, thereby facilitating the operation of the surgical staff and making better operation continuity for the surgical staff; and meanwhile, the smoke can be exhausted in time, the safety and continuity of the surgery are ensured, and the health of a patient and the surgical medical staff is guaranteed.

Further specifically, as shown in Fig. 1, in the automatic surgical smoke exhaust system according to the present disclosure, the automatic start/stop control unit 22 includes a working status collecting unit 221 and a control unit 222. The control unit 222 is configured to analyze working status information fed back by the working status collecting unit 221 and controls the start/stop of the smoke exhaust host 21. A specific structure and arrangement form of the working status collecting unit 221 are determined according to an actual discrimination method. The specific structures and arrangement forms of the working status collecting unit 221 will be described below with several different embodiments.

### Embodiment 1:

As shown in Fig. 1, this embodiment aims to use power transmitted from the power supply to the energy generator 13 to determined real-time working power of the energy instrument 12, so that the control unit 222 can control the smoke exhaust host 21 to perform synchronous start or intermittent start at a predetermined time according to the magnitude of the real-time working power of the energy instrument 12 and a working duration at this working power. For example, if standby power transmitted from the power supply to the energy generator 13 before the surgery is 90 W, 90 W may be set as a threshold value that the control unit 222 controls the smoke exhaust host 21 to start. That is, when the power transmitted from the power supply to the energy generator 13 is greater than 90 W, the smoke exhaust host 21 may be controlled to start for smoke exhaust. Apparently, it may be understood that in order to save energy, when the power transmitted from the power supply to the energy generator 13 is greater than 90 W but less than 200 W, the smoke exhaust host 21 may be controlled to start in an intermittent manner with different time intervals according to the magnitudes of the specific power to exhaust the smoke, and when the power transmitted from the power supply to the energy generator 13 is greater than or equal to 200 W, the smoke exhaust host 21 is controlled to start synchronously with the energy instrument 12 for smoke exhaust.

Therefore, the automatic surgical smoke exhaust system may be provided in such a way that the energy generator 13 is connected to the power supply, the working status collecting unit 221 is electrically connected between the energy generator 13 and the power supply, so that the control unit 222 can obtain a working status of the energy instrument 12 according to the power transmitted between the power supply and the energy generator 13. Specifically, the working status collecting unit 221 may be a set of voltammeters to measure voltages at both ends of the generator 13 and a current between the power supply and the energy generator 13, thereby obtaining the power transmitted from the power supply to the energy generator 13. Apparently, it may be understood that if the voltages at the ends of the energy generator 13 are constant, it is only necessary to measure the magnitude of the current between the power supply and the energy generator 13; and if the magnitude of the current between the power supply and the energy generator 13 is constant, it is only necessary to measure the voltages at the both ends of the energy generator 13. Preferably, the working status collecting unit 221 may also be a Hall sensor, which is connected between the power supply and the energy generator. Since the Hall sensor is small in size and low in weight, such design enables the surgical device 1 to be lightweight, which facilitates the operation by the surgical staff.

### Embodiment 2:

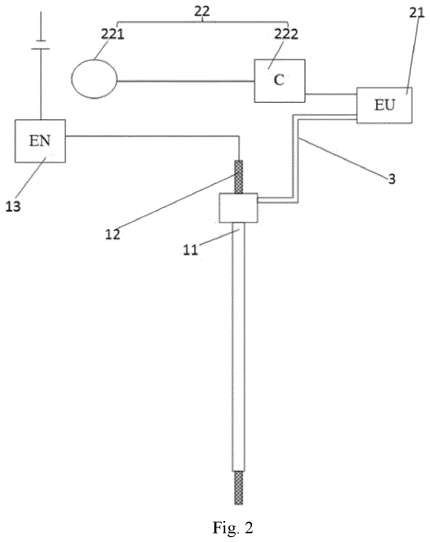

According to this embodiment, as shown in Fig. 2, the energy generator 13 is configured to emit no sound wave when standby and emit, in an intermittent manner with different time intervals according to the magnitudes of the specific power, sound waves of a set frequency when energy output is performed. Thus, the working status collecting unit 221 should be provided as an acoustic sensor, and the control unit 222 is embedded inside with a sound recognition module to recognize an interval time between two sound waves emitted by the energy generator 13 according to signals transmitted by the acoustic sensor, thereby determining whether the energy generator 13 outputs energy to the energy instrument 12 as well as the energy output power. For example, when the power of the energy output from the energy generator 13 to the energy instrument 12 is 0 W, i.e., an interval time between two detected sound waves is greater than 3 s, it is determined that there is no energy output from the energy generator 13, and thus there is no need to start the smoke exhaust host 21. When the power of the energy output from the energy generator 13 to the energy instrument 12 is 0 W to 180 W, i.e., an interval time between two detected sound waves is greater than 1 s but less than or equal to 3 s, the smoke exhaust host 21 is controlled to start intermittently at a predetermined time according to the magnitude of actual output power of the energy generator 13. When the power of the energy output from the energy generator 13 to the energy instrument 12 is greater than 180 W, i.e., an interval time between two detected sound waves is less than or equal to 1 s, the smoke exhaust host 21 is controlled to start synchronously with the energy instrument 12 for smoke exhaust.

### Embodiment 3:

According to this embodiment, as shown in Fig. 2, the energy generator 13 may further be provided with a light-emitting unit, and the light-emitting unit may be configured in such a way that a light color of emitted indicator light varies with the magnitude of the power of output energy. Thus, the working status collecting unit 221 should be provided as a light color recognition module, so that it is able to avoid interference of other lights in a surgical environment and determine the power of the energy output from the energy generator 13 to the energy instrument 12 according to different colors of the indicator light. For example, when the energy instrument 12 is standby, the light-emitting unit emits green light; when the energy instrument 12 runs at a low power, the light-emitting unit emits yellow light; and when the energy instrument 12 runs at a high power, the light-emitting unit emits red light. However, the recognition of color lights has high requirements for the configuration of the working status collecting unit 221, which is not conducive to cost saving. As such, the light-emitting unit may be preferably configured to be always turn-on when the energy instrument 12 is standby, and to flash at a set frequency when the energy instrument 12 runs. As such, the working status collecting unit 221 should be provided as a light intensity recognition module, so that the control unit 222 may determine the working power of the energy instrument 12 through a flash frequency, and thereby control the smoke exhaust host 21 to perform synchronous start or intermittent start at a predetermined time. The preferred solution has low configuration requirements for both the light-emitting unit and the working status collecting unit 221, which is conducive to cost saving.

### Embodiment 4:

In an embodiment of the automatic surgical smoke exhaust system according to the present disclosure, this embodiment aims to use a surgical site as well as a temperature at an acting area where the energy instrument 12 works on the surgical site to infer whether smoke is generated or to estimate the magnitude of an amount of the smoke, so that it is able to control the smoke exhaust host 21 to perform synchronous start or intermittent start at a predetermined time. For example, when a temperature at an acting area where the energy instrument 12 works at a surgical site is greater than or equal to 200°C, the smoke exhaust host 21 may be controlled to synchronously start for smoke exhaust; and when the temperature is less than 200°C, the smoke exhaust host 21 may be controlled to start intermittently at different time intervals according to the magnitudes of the specific power to exhaust smoke.

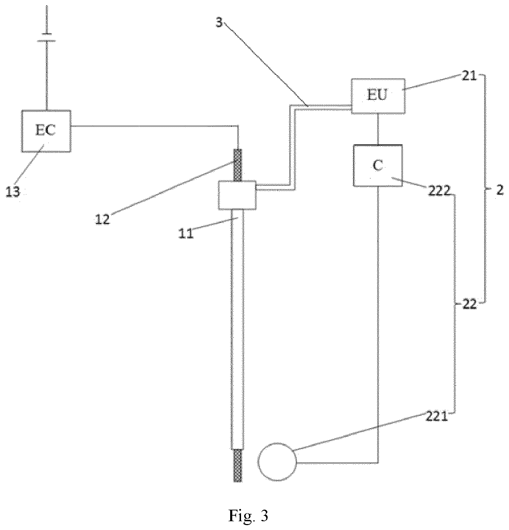

Therefore, as shown in Fig. 3, the working status collecting unit 221 may be provided as a temperature sensor, so that it is able to recognize the temperature at the acting area where the energy instrument 12 works on the surgical site.

### Embodiment 5:

In an embodiment of the automatic surgical smoke exhaust system according to the present disclosure, this embodiment aims to directly detect whether smoke is generated or how is an amount of the smoke when the energy instrument 12 acts at a surgical site, and thereby control the smoke exhaust host 21 to perform synchronous start or intermittent start at a predetermined time.

Therefore, as shown in Fig. 3, the working status collecting unit 221 may be provided as a smoke sensor, so that it is able to recognize a concentration of the smoke generated while the energy instrument 12 is acting at a surgical site. Specifically, multiple levels of numerical criteria for the concentration of the smoke may be set. For example, when a concentration of the smoke reaches 1 mg/m3 within 10 s, but is less than 10 mg/m3, the control unit 222 controls the smoke exhaust host 21 to intermittently start for smoke exhaust, and when the concentration of the smoke reaches 10 mg/m3 within 10 s, the control unit 222 controls the smoke exhaust host 21 to start synchronously with the energy instrument 12 for smoke exhaust. The technical solution can determine the concentration of the smoke in the space of surgery more directly and accurately.

### Embodiment 6:

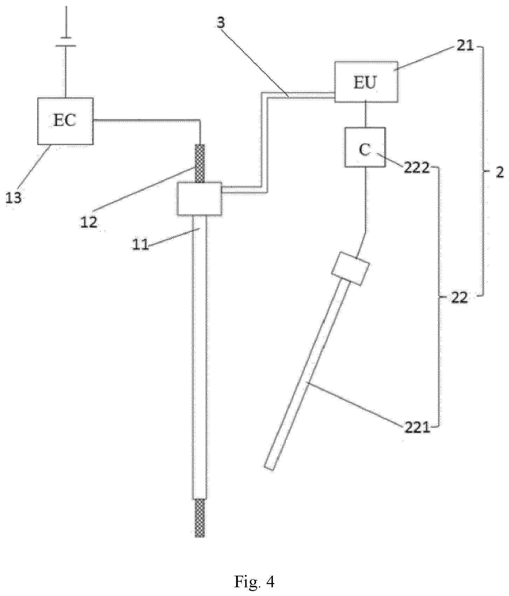

Minimally invasive technology is usually applied on surgeries of the intracorporal parts, such as thoracic cavities and abdominal cavities of patients, and such a surgery for an intracorporal part of the patient usually requires the use of an image acquisition device and an image display device (e.g., an endoscope) to observe or perform operation at a surgical site. Therefore, as shown in Fig. 4, the image acquisition device and the image display device may be directly used as the working status collecting unit 221, and the control unit 222 is embedded with an image processing module so that images captured by the image acquisition device are analyzed by the image processing module. In this way, whether the smoke in the space of surgery would obstruct a surgical field of view can be determined through analysis of the image processing module, and the control unit 222 can automatically control the start/stop of the smoke exhaust host 21 to achieve automatic smoke exhaust, thereby facilitating surgical operation by an operator. Alternatively, it is possible for human eyes to acquire a picture more intuitively from the image display device, so that it is able to perform active control on the start/stop of the smoke exhaust host 21 according to subjective judgment of the operator, and accordingly adapt to the habits of various operators.

The preferred embodiments of the present disclosure are described in detail above in conjunction with the drawings. However, the present disclosure is not limited to the specific details in the above embodiments, and a variety of simple variants of the technical solutions of the present disclosure may be performed within the scope of the technical conception of the present disclosure, and all of which fall within the scope of protection of the present disclosure.

Additionally, it is to be noted that all the specific technical features described in the above specific embodiments may be combined in any suitable manner without conflicts. In order to avoid unnecessary repetition, various possible combinations are not described separately in the present disclosure.

Additionally, the various different embodiments of the present disclosure may also be combined arbitrarily as long as they do not deviate from the idea of the present disclosure, and they shall also be regarded as the contents disclosed in the present disclosure.

## Claims

1. An automatic surgical smoke exhaust system, comprising a smoke exhaust channel (11) and a surgical smoke exhaust device (2), wherein the smoke exhaust channel (11) can guide smoke generated by an energy instrument (12) at a surgical site to the surgical smoke exhaust device (2); the surgical smoke exhaust device (2) comprises a smoke exhaust host (21) and an automatic start/stop control unit (22) electrically connected to the smoke exhaust host (21); the smoke exhaust channel (11) is connected to the smoke exhaust host (21) by means of a pipeline (3); the automatic start/stop control unit (22) can detect a working status of the energy instrument (12), and control start/stop of the smoke exhaust host (21) according to working status information.

2. The automatic surgical smoke exhaust system according to claim 1, wherein the automatic start/stop control unit (22) comprises a working status collecting unit (221) and a control unit (222), and the control unit (222) is configured to analyze the working status information fed back by the working status collecting unit (221) and controls the start/stop of the smoke exhaust host (21).

3. The automatic surgical smoke exhaust system according to claim 1, wherein the energy instrument (12) is inserted into the smoke exhaust channel (11), and a working end of the energy instrument (11) extends out of the smoke exhaust channel (11).

4. The automatic surgical smoke exhaust system according to claim 2, wherein the working status collecting unit (221) is electrically connected between an energy generator (13) and a power supply, and the control unit (222) can obtain the working status of the energy instrument (12) according to transmission power between the power supply and the energy generator (13).

5. The automatic surgical smoke exhaust system according to claim 4, wherein the working status collecting unit (221) is a Hall sensor.

6. The automatic surgical smoke exhaust system according to claim 4, wherein the working status collecting unit (221) is an acoustic sensor, so that it is able to detect sound waves intermittently emitted by the energy generator (13) according to a magnitude of output energy power thereof.

7. The automatic surgical smoke exhaust system according to claim 4, wherein the working status collecting unit (221) is a light color recognition module, so that it is able to detect indicator light of different colors emitted by a light-emitting unit on the energy generator (13) according to magnitudes of output energy power of the energy generator (13); or the working status collecting unit (221) is a light intensity recognition module, so that it is able to detect indicator light of different brightnesses emitted by the light-emitting unit on the energy generator (13) according to magnitudes of output energy power of the energy generator (13).

8. The automatic surgical smoke exhaust system according to claim 4, wherein the working status collecting unit (221) is a temperature sensor, so that it is able to recognize a temperature at an acting area where the energy instrument (12) works at the surgical site.

9. The automatic surgical smoke exhaust system according to claim 4, wherein the working status collecting unit (221) is a smoke sensor, so that it is able to recognize a concentration of the smoke generated while the energy instrument (12) is working at the surgical site.

10. The automatic surgical smoke exhaust system according to claim 2, wherein the working status collecting unit (221) comprises an image acquisition device and an image display device, the image acquisition device can capture the surgical site, and the control unit (222) is embedded with an image processing module so that it is able to recognize smoke in a picture captured by the image acquisition device.
